# EUROPEAN PATENT SPECIFICATION

(11) **EP 3 045 441 B1**
(45) Date of publication and mention of the grant of the patent: **18.03.2020**
(21) Application number: 16150996.3
(22) Date of filing: 13.01.2016
(51) Int. Cl.: C07C 17/25, C07C 17/42, C07C 21/18

(54) **PROCESS FOR PRODUCING FLUORINE-CONTAINING OLEFIN**
VERFAHREN ZUR HERSTELLUNG EINES FLUORHALTIGEN OLEFINS
PROCÉDÉ DE PRODUCTION D'OLÉFINE CONTENANT DU FLUOR

(30) Priority: 13.01.2015 JP 2015004101
(43) Date of publication of application: 20.07.2016
(73) Proprietor: DAIKIN INDUSTRIES, LTD., Osaka-shi, Osaka 530-8323 (JP)
(72) Inventor: Ohkubo, Shun, Osaka, 530-8323 (JP); Karube, Daisuke, Osaka, 530-8323 (JP)
(74) Representative: Hoffmann Eitle

(56) References cited:
- WO-A1-2012/098420
- US-A1- 2011 160 497

## Description

### Technical Field

The present invention relates to a process for producing a fluorine-containing olefin.

### Background Art

Fluoroolefins represented by formula CF₃(CX₂)ₙCF=CH₂, formula CF₃(CX₂)ₙCH=CHF, or the like, are compounds with a structure useful in functional materials, solvents, refrigerants, foaming agents, and monomers for functional polymers or starting materials for such monomers. For example, fluoroolefins are used as monomers for modifying ethylene-tetrafluoroethylene copolymers. Of the above-listed fluoroolefins, a compound (HFO-1234yf) represented by CF₃CF=CH₂ and a compound (HFO-1234ze) represented by CF₃CH=CHF show increasing promise as a refrigerant compound with a low global-warming potential.

One of the processes reported for producing fluoroolefins represented by the formulae has been a process that employs a reaction, in the presence of a catalyst, between a chlorine-containing alkane or chlorine-containing alkene, i.e., a starting material, having the same carbon number as the target fluoroolefin, and a fluorinating agent such as anhydrous hydrogen fluoride (see Patent Literature 1 below).

In particular, HFO-1234yf, which has attracted attention as a refrigerant with a low global-warming potential, is produced through a gas-phase fluorination continuous reaction of a chlorine-containing olefin such as CF₃CCl=CH₂ (HCFO-1233xf) in the presence of a catalyst. Patent Literature 1 discloses this process accompanied by oxidized gas, such as oxygen, to reduce the deterioration of the catalyst, which occurs during a continuous long-term reaction. Patent Literature 2 proposes the use of a chromium catalyst in which Ni is present together with Cr in the process for producing HFO-1234yf through a gas-phase fluorination continuous reaction of a chlorine-containing olefin such as HCFO-1233xf in the presence of a catalyst, and the Examples demonstrate the use of a catalyst containing Ni and Cr in a molar ratio of 50:50.

However, the use of oxygen to prevent the deterioration of a catalyst is disadvantageous in regards to the generation of CO₂ resulting from a side reaction in which the chlorine-containing compound (i.e., starting material) burns, thus decreasing the selectivity of the target product fluorine-containing olefin.

Hence, there remains a need to develop a process for producing a fluorine-containing olefin by reacting, in the presence of oxygen and a catalyst, anhydrous hydrogen fluoride with a chlorine-containing alkane or chlorine-containing alkene, i.e., a starting material, while sufficiently reducing the deterioration of the catalyst in the presence of oxygen and maintaining the conversion of the starting material within a preferable range, further reducing the amount of byproduct CO₂.

### Citation List

### Patent Literature

PTL 1: US2011-0160497A
PTL 2: WO2012/098420

### Summary of Invention

### Technical Problem

The present invention was completed in view of the above-described status quo of the prior art, and the primary object is to provide a process for producing a fluorine-containing olefin by reacting, in the presence of oxygen and a catalyst, anhydrous hydrogen fluoride with a chlorine-containing alkane or chlorine-containing alkene, i.e., a starting material, sufficiently reducing the deterioration of the catalyst in the presence of oxygen while maintaining the conversion of the starting material within a preferable range, further reducing the amount of byproduct CO₂.

### Solution to Problem

The present inventors conducted extensive research to achieve the object, and found that when a reaction is carried out using a catalyst in which a specific metal element M is present together with chromium in a specific molar ratio, the deterioration of the catalyst is sufficiently suppressed in the presence of oxygen while the conversion of the starting material is maintained within a preferable range, further reducing the amount of byproduct CO₂. The inventors then completed the present invention.

Specifically, the present invention provides the following process for producing a fluorine-containing olefin.
Item 1.
   A process for producing a fluorine-containing olefin represented by formula (7): CF₃CA=CHB wherein one of A and B is F or Cl and the other is H,
   the process comprising reacting, in the presence of oxygen and a fluorination catalyst, anhydrous hydrogen fluoride with at least one chlorine-containing compound selected from the group consisting of
   a chlorine-containing alkane represented by formula (1) CX₃CClYCH₂Z wherein each X independently represents F or Cl, Y is H or F, when Y is H, Z is Cl or F, and when Y is F, Z is H;
   a chlorine-containing alkane represented by formula (2) CX₃CH₂CHX₂ wherein each X independently represents F or Cl, and at least one X is Cl;
   a chlorine-containing alkene represented by formula (3) CX₃CCl=CH₂ wherein each X independently represents F or Cl;
   a chlorine-containing alkene represented by formula (4) CX₃CH=CHX wherein each X independently represents F or Cl, and at least one X is Cl;
   a chlorine-containing alkene represented by formula (5) CH₂XCCl=CX₂ wherein each X independently represents F or Cl; and
   a chlorine-containing alkene represented by formula (6) CHX₂CH=CX₂ wherein each X independently represents F or Cl,
   the fluorination catalyst being a catalyst in which at least one metal element M selected from the group consisting of indium, gallium, cobalt, copper, nickel, zinc, and aluminum is present together with chromium, wherein the molar ratio of the at least one metal element M to the chromium is 96:4 to 65:35, and wherein the metal element M comprises crystalline nickel fluoride.
Item 2.
   The process according to Item 1 wherein the fluorination catalyst is a catalyst in which nickel fluoride is present together with chromium oxide.
Item 3.
   The process according to any one of Items 1 to 2 wherein the molar ratio of the at least one metal element M to the chromium is 90:10 to 75:25.
Item 4.
   The process according to any one of Items 1 to 3 wherein the fluorination catalyst is not supported on a carrier.
Item 5.
   The process according to any one of Items 1 to 3 wherein the fluorination catalyst is supported on a carrier selected from the group consisting of nickel fluoride, chromium fluoride, aluminum fluoride, fluorinated alumina, fluorinated activated carbon, and graphite carbon.
Item 6.
   The process according to any one of Items 1 to 5 wherein the fluorination catalyst has a specific surface area of 30 m²/g or more.
Item 7.
   The process according to any one of Items 1 to 6 wherein the chlorine-containing compound is at least one member selected from the group consisting of HCC-240db, HCO-1230xa, HCO-1230xf, HCFC-243db, HCFC-242dc, HCFO-1231xf, HCFO-1232xf, and HCFO-1233xf, and the fluorine-containing olefin to be obtained is HCFO-1233xf or HFO-1234yf.
Item 8.
   The process according to any one of Items 1 to 6 wherein the chlorine-containing compound is at least one member selected from the group consisting of HCC-240fa, HCO-1230za, HCFC-243fa, and HCFO-1233zd, and the fluorine-containing olefin to be obtained is HCFO-1233zd or HFO-1234ze.

### Advantageous Effects of Invention

The process for producing a fluorine-containing olefin according to the present invention can provide a target fluorine-containing olefin from a chlorine-containing compound, i.e., a starting material, represented by a specific formula at an excellent starting material conversion, while reducing the amount of byproduct CO₂. Because a reaction proceeds in the presence of oxygen in this process, even a continuous reaction reduces the catalytic activity to a lesser extent.

Thus, the production process according to the present invention can continuously produce a fluorine-containing olefin efficiently without conducting cumbersome treatments such as catalyst replacement and recycling treatment. Thus, the production process according to the present invention is advantageous in regards to the production of fluorine-containing olefins on an industrial scale.

### Description of Embodiments

The following describes in detail the process for producing a fluorine-containing olefin according to the present invention.

Table 1 shows the specific names, structures, and chemical names of compounds that fall within the scope of formulae (1) to (7) for use in this specification.

**Table 1**

| Formula | Name | Structure | Chemical Name |
|---|---|---|---|
| (1) | HCFC-243db | CF₃CHClCH₂Cl | 2,3-dichloro-1,1,1-trifluoropropane |
| (1) | HCFC-242dc | CF₂ClCHClCH₂Cl | 1,2,3-trichloro-1,1-difluoropropane |
| (1) | HCFC-241dc | CFCl₂CHClCH₂Cl | 1,1,2,3-tetrachloro-1-fluoropropane |
| (1) | HCC-240db | CCl₃CHClCH₂Cl | 1,1,1,2,3-pentachloropropane |
| (2) | HCC-240fa | CCl₃CH₂CHCl₂ | 1,1,1,3,3-pentachloropropane |
| (2) | HCFC-243fa | CF₃CH₂CHCl₂ | 3,3-dichloro-1,1,1-trifluoropropane |
| (3), (7) | HCFO-1233xf | CF₃CCl=CH₂ | 2-chloro-3,3,3-trifluoropropene |
| (3) | HCO-1230xf | CCl₃CCl=CH₂ | 2,3,3,3-tetrachloropropene |
| (3) | HCFO-1231xf | CFCl₂CCl=CH₂ | 2,3,3-trichloro-3-fluoropropene |
| (3) | HCFO-1232xf | CF₂ClCCl=CH₂ | 2,3-dichloro-3,3-difluoropropene |
| (4), (7) | HCFO-1233zd | CF₃CH=CHCl | 1-chloro-3,3,3-trifluoropropene |
| (4) | HCO-1230zd | CCl₃CH=CHCl | 1,3,3,3-tetrachloropropene |
| (5) | HCO-1230xa | CH₂ClCCl=CCl₂ | 1,1,2,3-tetrachloropropene |
| (6) | HCO-1230za | CHCl₂CH=CCl₂ | 1,1,3,3-tetrachloropropene |
| (7) | HFO-1234yf | CF₃CF=CH₂ | 2,3,3,3-tetrafluoropropene |
| (7) | HFO-1234ze | CF₃CH=CHF | 1,3,3,3-tetrafluoropropene |

### (I) Starting Compounds

The present invention uses at least one chlorine-containing compound selected from the group consisting of the following compounds represented by formulae (1) to (6). A chlorine-containing alkane represented by formula (1): CX₃CClYCH₂Z wherein each X independently represents F or Cl; Y is H or F; when Y is H, Z is Cl or F; and when Y is F, Z is H A chlorine-containing alkane represented by formula (2): CX₃CH₂CHX₂ wherein each X independently represents F or Cl, and at least one X is Cl
A chlorine-containing alkene represented by formula (3): CX₃CCl=CH₂ wherein each X independently represents F or Cl A chlorine-containing alkene represented by formula (4): CX₃CH=CHX wherein each X independently represents F or Cl, and at least one X is Cl
A chlorine-containing alkene represented by formula (5): CH₂XCCl=CX₂ wherein each X independently represents F or Cl A chlorine-containing alkene represented by formula (6): CHX₂CH=CX₂ wherein each X independently represents F or Cl

A reaction of these starting material chlorine-containing compounds with anhydrous hydrogen fluoride in accordance with the below-described conditions can provide target fluorine-containing olefins represented by formula (7): CF₃CA=CHB wherein one of A and B is F or Cl and the other is H.

Of the starting materials above, specific examples of chlorine-containing alkane represented by formula (1): CX₃CClYCH₂Z include 2,3-dichloro-1,1,1-trifluoropropane (CF₃CHClCH₂Cl(HCFC-243db)), 1,2,3-trichloro-1,1-difluoropropane (CF₂ClCHClCH₂Cl(HCFC-242dc)), 1,1,2,3-tetrachloro-1-fluoropropane (CFCl₂CHClCH₂Cl(HCFC-241dc)), and 1,1,1,2,3-pentachloropropane (CCl₃CHClCH₂Cl(HCC-240db)).

Specific examples of chlorine-containing alkane represented by formula (2): CX₃CH₂CHX₂ include 1,1,1,3,3-pentachloropropane (CCl₃CH₂CHCl₂(HCC-240fa)), and 3,3-dichloro-1,1,1-trifluoropropane (CF₃CH₂CHCl₂(HCFC-243fa)).

Specific examples of chlorine-containing alkene represented by formula (3): CX₃CCl=CH₂ include 2-chloro-3,3,3-trifluoropropene (CF₃CCl=CH₂(HCFO-1233xf)), 2,3,3,3-tetrachloropropene (CCl₃CCl=CH₂(HCO-1230xf)), 2,3,3-trichloro-3-fluoropropene (CFCl₂CCl=CH₂(HCFO-1231xf), and 2,3-dichloro-3,3-difluoropropene (CF₂ClCCl=CH₂(HCFO-1232xf)).

Specific examples of chlorine-containing alkene represented by formula (4): CX₃CH=CHX include 1-chloro-3,3,3-trifluoropropene (CF₃CH=CHCl(HCFO-1233zd)), and 1,3,3,3-tetrachloropropene (CCl₃CH=CHCl(HCO-1230zd)).

Specific examples of chlorine-containing alkene represented by formula (5): CH₂XCCl=CX₂ include 1,1,2,3-tetrachloropropene (CH₂ClCCl=CCl₂(HCO-1230xa)).

Specific examples of chlorine-containing alkene represented by formula (6): CHX₂CH=CX₂ include 1,1,3,3-tetrachloropropene (CHCl₂CH=CCl₂(HCO-1230za)).

In the present invention, these starting materials can be used singly, or in combination of two or more.

### (II) Reaction Process

The production process according to the present invention comprises a reaction of the above-listed starting compounds with hydrogen fluoride in the presence of oxygen and a specific fluorination catalyst. The production process according to the present invention can sufficiently reduce deterioration of the catalyst because of the use of oxygen (oxygen gas) and can maintain the conversion of the starting compound in a preferable range because of the use of the specific fluorination catalyst, further reducing the amount of byproduct CO₂.

Oxygen is preferably supplied in an amount of about 0.005 to 0.2 moles, and more preferably about 0.01 to 0.15 moles per mole of a chlorine-containing compound for use as a starting material. In the present invention, maintaining the reaction temperature in the below-described range helps the oxygen to sufficiently reduce the deterioration of a catalyst.

The procedure for conducting the reaction in the presence of oxygen is not particularly limited. Oxygen may be typically supplied to a reactor together with a chlorine-containing compound used as a starting material, or may also be supplied to a rector after being dissolved in the chlorine-containing compound.

The catalyst for use in the production process according to the present invention is a fluorination catalyst in which at least one metal element M selected from the group consisting of indium, gallium, cobalt, copper, nickel, zinc, and aluminum is present together with chromium in a metal element M:chromium molar ratio of 96:4 to 65:35, and wherein the metal element M comprises crystalline nickel fluoride. Of these, catalysts in which at least one metal element M and chromium are present in a molar ratio of 90:10 to 75:25 are preferable. In the present invention, the use of the specific fluorination catalyst helps the oxygen to sufficiently reduce the deterioration of the catalyst, and to reduce the amount of byproduct CO₂ generated by the use of oxygen.

Any catalysts can be used as long as the at least one metal element M is present together with chromium in the catalysts and the metal element M comprises crystalline nickel fluoride. Catalysts containing chromium in its chromium oxide form are preferable.

Specifically, preferable catalysts are those containing chromium oxide highly dispersed in the crystalline structure of the at least one metal element M comprising crystalline nickel fluoride. These catalysts preferably contain chromium oxide in an amount of about 4 to 35 mol%, and more preferably about 10 to 25 mol% based on the total metal amount of the at least one metal element M comprising crystalline nickel fluoride and chromium oxide.

In the present invention, the at least one metal element M comprises crystalline nickel fluoride, and preferably is crystalline nickel fluoride. Of the catalysts in which nickel fluoride is present together with chromium oxide, the most preferable catalysts are those in which chromium oxide is highly dispersed in the crystalline structure of nickel fluoride.

Chromium oxide for use in the catalyst is particularly preferably at least one member selected from the group consisting of chromium oxide and fluorinated chromium oxide. Examples of chromium oxide or fluorinated chromium oxide for use include crystalline chromium oxide and amorphous chromium oxide. The composition of chromium oxide is not particularly limited; however, a preferable chromium oxide for use is represented by composition formula CrOₘ wherein 1.5<m<3, and more preferable chromium oxide is represented by composition formula CrOₘ wherein 2<m<2.75.

Fluorinated chromium oxide can be obtained, for example, by fluorinating chromium oxide with hydrogen fluoride (HF treatment). The fluorination temperature may be, for example, about 100 to 460°C. Chromium oxide can be fluorinated, for example, by supplying anhydrous hydrogen fluoride to a reactor charged with chromium oxide.

Nickel fluoride may be mixed with chromium oxide, or a composite oxide of chromium and nickel may be fluorinated.

In the production process of the present invention, a reaction proceeds in the presence of hydrogen fluoride, and thus fluorination of a catalyst is thought to proceed during the reaction even when a fluorination treatment is not conducted beforehand.

The degree of fluorination of chromium oxide is not particularly limited. For example, fluorinated chromium oxide with a fluorine content of about 5 to 30 wt% is preferably used.

The specific surface area of fluorination catalyst for use in the present invention is not limited, but, for example, is preferably 30 m²/g or more, and more preferably 50 m²/g or more.

The fluorination catalyst for use in the present invention can be in any form, such as powder or pellet, as long as the form is suitable for the reaction. In particular, catalysts in pellet form are preferable.

The fluorination catalyst for use in the present invention may not be supported on a carrier, or may optionally be supported on a carrier. The carrier is not particularly limited, but is preferably at least one member selected from the group consisting of nickel fluoride, chromium fluoride, aluminum fluoride, fluorinated alumina, fluorinated activated carbon, and graphite carbon.

The procedure for using the catalyst is not particularly limited, as long as the starting compound is used in a manner in which it sufficiently comes into contact with the catalyst. Examples include a procedure comprising immobilizing a catalyst in a reactor to form a catalyst layer, and a procedure comprising dispersing a catalyst in a fluid bed.

Anhydrous hydrogen fluoride may be typically supplied to a reactor together with a starting compound. The amount of anhydrous hydrogen fluoride for use is not particularly limited, but is preferably about 4 moles or more, and more preferably about 8 moles or more per mole of the chlorine-containing compound used as a starting material from the standpoint of high selectivity of the target fluorine-containing olefin.

The upper limit of the amount of anhydrous hydrogen fluoride for use is not particularly limited. Hydrogen fluoride, when added in an excess amount, does not significantly affect the selectivity and conversion, but reduces the productivity because of an increase in the amount of hydrogen fluoride separated off in purification. It is thus typically preferable to use anhydrous hydrogen fluoride in an amount of about 100 moles or less, and more preferably in an amount of about 50 moles or less, per mole of a chlorine-containing compound used as a starting material.

In the production process of the present invention, the reaction temperature is not limited, and can be suitably adjusted depending on the type of starting compound for use. However, a preferable reaction temperature is typically in the range of 200 to 450°C.

For example, when 2-chloro-3,3,3-trifluoropropene (HCFO-1233xf) is used as a starting material, the reaction temperature is preferably in the range of about 300 to 450°C, and more preferably about 325 to 400°C in order to maintain the conversion of the starting material and the selectivity of the target product within a preferable range. In particular, the amount of oxygen for use is preferably about 0.01 to 0.5 moles, and more preferably about 0.02 to 0.15 moles per mole of 2-chloro-3,3,3-trifluoropropene (HCFO-1233xf).

The pressure for the reaction is not particularly limited, and the reaction can be carried out under reduced pressure, ordinary pressure, or increased pressure. Typically, the reaction may be carried out under a pressure near atmospheric pressure (0.1 MPa), but can also be carried out smoothly under a reduced pressure of less than 0.1 MPa. The reaction may also be carried out under a pressure increased to a degree at which the starting material is not liquefied.

In a specific example of embodiments of the present invention, a tubular flow-through reactor is charged with a fluorination catalyst, and then a chlorine-containing compound used as a starting material, anhydrous hydrogen fluoride, and oxygen are introduced to the reactor.

The starting compound should be in a gaseous state when brought into contact with anhydrous hydrogen fluoride, but may be in a liquid state when first supplied to the reactor. For example, the starting compound, when in a liquid state at room temperature and ordinary pressure, may be vaporized with a vaporizer (vaporization region) and then allowed to pass through a preheating region to be supplied to a mixing region where the starting compound comes into contact with anhydrous hydrogen fluoride. This procedure enables a reaction to proceed in a gas phase. Alternatively, the starting compound in a liquid state may be supplied to a reactor, while a catalyst layer placed in the reactor is being heated to or above the vaporization temperature of the starting compound. Then, the starting compound, when having reached the region for reaction with hydrogen fluoride, may be vaporized to carry out a reaction.

The above-described starting materials may be directly supplied to a reactor, or may be supplied in the presence of a gas inert to the starting materials and catalyst, such as nitrogen, helium, and argon. The concentration of the inert gas may be about 0 to 80 mol% based on the total amount of the inert gas and gaseous components (i.e., the chlorine-containing compound, anhydrous hydrogen fluoride, and chlorine gas) introduced into the reactor.

The contact time is not limited. However, excessively short contact time may result in insufficient conversion in the reaction, and excessively long contact time may result in an increase in byproduct production. Thus, the contact time may be suitably determined while taking these effects into account. For example, the contact time represented by W/F₀, the ratio of the amount of catalyst supplied W (g) to the total flow of the starting material gas introduced into the reaction system Fo (0°C, flow rate at 0.1 MPa: mL/sec), is preferably in the range of about 0.5 to 70 g·sec/mL, and more preferably about 1 to 50 g·sec/mL. The total flow of the starting material gas as used herein refers to the total of the flow of the chlorine-containing compound, anhydrous hydrogen fluoride, and oxygen, with the flow of inert gas when added.

### (III) Reaction Product

The process described above can produce a target fluorine-containing olefin represented by formula (7): CF₃CA=CHB wherein one of A and B is F or Cl and the other is H at a high selectivity from a chlorine-containing compound represented by formulae (1) to (6), i.e., a starting material. Even when the reaction continues, the process can maintain high selectivity for a long period of time with less decrease in the catalytic activity.

Specific examples of fluorine-containing olefins represented by formula (7) include 2,3,3,3-tetrafluoropropene (HFO-1234yf) represented by formula: CF₃CF=CH₂, and 1,3,3,3-tetrafluoropropene (HFO-1234ze) represented by formula: CF₃CH=CHF.

For example, the use of CF₃CHClCH₂Cl(HCFC-243db), CF₂ClCHClCH₂Cl (HCFC-242dc), CFCl₂CHClCH₂Cl (HCFC-241dc), CF₃CCl=CH₂ (HCFO-1233xf), CF₂ClCCl=CH₂ (HCFO-1232xf), CFCl₂CCl=CH₂ (HCFO-1231xf), CH₂ClCCl=CCl₂ (HCO-1230xa), CCl₃CCl=CH₂ (HCO-1230xf), or the like as a starting material provides 2,3,3,3-tetrafluoropropene (HFO-1234yf) as a major component. The use of CCl₃CH₂CHCl₂ (HCC-240fa), CHCl₂CH=CCl₂ (HCO-1230za), CF₃CH=CHC (HCFO-1233zd), CCl₃CH=CHCl (HCO-1230zd)), or the like as a starting material provides 1,3,3,3-tetrafluoropropene (HFO-1234ze) as a major component.

The process according to the present invention separates and collects the generated product at the reactor outlet by a technique such as distillation to thereby obtain a target fluorine-containing olefin represented by formula (7).

The 1,1,1,2,2-pentafluoropropane (HFC-245cb), a major component of the byproduct contained in the generated product, can be easily converted into 2,3,3,3-tetrafluoropropene (HFO-1234yf) by dehydrofluorination, and thus can be effectively used as a useful compound.

### Examples

The following Examples and Comparative Examples describe the present invention in detail. However, the present invention is not limited to these Examples.

### Example 1

A metal reactor tube was charged with 5.7 g of a fluorination catalyst (metal molar ratio: Cr/(Ni+Cr)=0.14, specific surface area: 32 cm²/g) obtained by highly dispersing chromium oxide in the crystalline structure of nickel fluoride.

Subsequently, the temperature of the reactor tube was increased to 350°C. Hydrogen fluoride gas and oxygen gas were supplied to the reactor at a flow rate of 35 Nml/min and 0.35 Nml/min, respectively, and maintained for 15 hours. Thereafter, 2-chloro-3,3,3-trifluoropropene (CF₃CCl=CH₂(HCFO-1233xf)) gas was supplied to the reactor tube at a flow rate of 3.5 Nml/min. About 2 hours later, the effluent gas was first sampled from the reactor and analyzed by gas chromatography.

Table 2 shows the results. Since 1,1,1,2,2-pentafluoropropane (HFC-245cb) in the generated product is a useful compound that can be converted into 2,3,3,3-tetrafluoropropene (HFO-1234yf) by dehydrofluorination, Table 2 also shows the total selectivity of HFO-1234yf and HFC-245cb as a value including the selectivity of the useful compound.

### Example 2

The fluorination reaction of Example 1 was repeated except that the metal molar ratio of the fluorination catalyst was changed to 0.21. Table 2 shows the results.

### Example 3

The fluorination reaction of Example 1 was repeated except that the metal molar ratio of the fluorination catalyst was changed to 0.04. Table 2 shows the results.

### Comparative Example 1

The fluorination reaction of Example 1 was repeated except that the fluorination catalyst was replaced with chromium oxide Cr₂O₃. Table 2 shows the results.

### Comparative Example 2

The fluorination reaction of Example 1 was repeated except that the fluorination catalyst was replaced with nickel fluoride NiF₂. Table 2 shows the results.

**Table 2**

| | | Example 1 | Example 2 | Example 3 | Comparative Example 1 | Comparative Example 2 |
|---|---|---|---|---|---|---|
| Reaction Temperature (°C) | | 365 | 365 | 365 | 365 | 365 |
| Contact Time (W/F₀ g/Nml·sec) | | 9 | 9 | 9 | 9 | 9 |
| Hydrogen Fluoride: Starting Material (Molar Ratio) | | 10:1 | 10:1 | 10:1 | 10:1 | 10:1 |
| Type of Catalyst Deterioration Inhibitor | | Oxygen | Oxygen | Oxygen | Oxygen | Oxygen |
| Mol% Based on Starting Material | | 10% | 10% | 10% | 10% | 10% |
| 1233xf Conversion (GC%) | | 16 | 4 | 6 | 18 | 0.5 |
| Selectivity (yield %) | 1234yf+245cb | 86 | 82 | 68 | 85 | Trace |
| | CO₂ | 0.8 | 0.9 | 1.2 | 2.2 | Trace |

As is clear from the results shown in Table 2, despite the oxygen contained in the reaction system, the amount of byproduct CO₂ was sufficiently reduced in Examples 1 to 3, which employed a fluorination catalyst obtained by highly dispersing chromium oxide in the crystalline structure of nickel fluoride in a predetermined metal molar ratio (Cr/(Ni+Cr)=0.04 to 0.35). In particular, Example 1 showed excellent conversion of the starting compound while maintaining excellent selectivity of the target product, further sufficiently reducing the amount of byproduct CO₂.

In contrast, although Comparative Example 1, which employed crystallized chromium as a fluorination catalyst, exhibited excellent selectivity of the target product, it showed a byproduct CO₂ amount of as high as 2.2.

## Claims

1. A process for producing a fluorine-containing olefin represented by formula (7): CF₃CA=CHB wherein one of A and B is F or Cl and the other is H,
the process comprising reacting, in the presence of oxygen and a fluorination catalyst, anhydrous hydrogen fluoride with at least one chlorine-containing compound selected from the group consisting of
a chlorine-containing alkane represented by formula (1) CX₃CClYCH₂Z wherein each X independently represents F or Cl, Y is H or F, when Y is H, Z is Cl or F, and when Y is F, Z is H;
a chlorine-containing alkane represented by formula (2) CX₃CH₂CHX₂ wherein each X independently represents F or Cl, and at least one X is Cl;
a chlorine-containing alkene represented by formula (3) CX₃CCl=CH₂ wherein each X independently represents F or Cl;
a chlorine-containing alkene represented by formula (4) CX₃CH=CHX wherein each X independently represents F or Cl, and at least one X is Cl;
a chlorine-containing alkene represented by formula (5) CH₂XCCl=CX₂ wherein each X independently represents F or Cl; and
a chlorine-containing alkene represented by formula (6) CHX₂CH=CX₂ wherein each X independently represents F or Cl,
the fluorination catalyst being a catalyst in which at least one metal element M selected from the group consisting of indium, gallium, cobalt, copper, nickel, zinc, and aluminum is present together with chromium, wherein the molar ratio of the at least one metal element M to the chromium is 96:4 to 65:35, and wherein the metal element M comprises crystalline nickel fluoride.

2. The process according to Claim 1 wherein the fluorination catalyst is a catalyst in which nickel fluoride is present together with chromium oxide.

3. The process according to any one of Claims 1 to 2 wherein the molar ratio of the at least one metal element M to the chromium is 90:10 to 75:25.

4. The process according to any one of Claims 1 to 3 wherein the fluorination catalyst is not supported on a carrier.

5. The process according to any one of Claims 1 to 3 wherein the fluorination catalyst is supported on a carrier selected from the group consisting of nickel fluoride, chromium fluoride, aluminum fluoride, fluorinated alumina, fluorinated activated carbon, and graphite carbon.

6. The process according to any one of Claims 1 to 5 wherein the fluorination catalyst has a specific surface area of 30 m²/g or more.

7. The process according to any one of Claims 1 to 6 wherein the chlorine-containing compound is at least one member selected from the group consisting of HCC-240db, HCO-1230xa, HCO-1230xf, HCFC-243db, HCFC-242dc, HCFO-1231xf, HCFO-1232xf, and HCFO-1233xf, and the fluorine-containing olefin to be obtained is HCFO-1233xf or HFO-1234yf.

8. The process according to any one of Claims 1 to 7 wherein the chlorine-containing compound is at least one member selected from the group consisting of HCC-240fa, HCO-1230za, HCFC-243fa, and HCFO-1233zd, and the fluorine-containing olefin to be obtained is HCFO-1233zd or HFO-1234ze.

## Patentansprüche

1. Verfahren zur Herstellung eines fluorhaltigen Olefins, dargestellt durch Formel (7): CF₃CA=CHB, worin eines von A und B F oder Cl ist und das andere H ist,
wobei das Verfahren das Umsetzen, in der Gegenwart von Sauerstoff und einem Fluorierungskatalysator, von wasserfreiem Wasserstofffluorid mit zumindest einer chlorhaltigen Verbindung umfasst, ausgewählt aus der Gruppe, bestehend aus
einem durch die Formel (1) CX₃CClYCH₂Z dargestellten chlorhaltigen Alkan, worin jedes X unabhängig F oder Cl darstellt, Y H oder F ist, wenn Y H ist, Z Cl oder F ist, und wenn Y F ist, Z H ist;
einem durch die Formel (2) CX₃CH₂CHX₂ dargestellten chlorhaltigen Alkan, worin jedes X unabhängig F oder Cl darstellt und zumindest ein X Cl ist;
einem durch die Formel (3) CX₃CCl=CH₂ dargestellten chlorhaltigen Alken, worin jedes X unabhängig F oder Cl ist;
einem durch die Formel (4) CX₃CH=CHX dargestellten chlorhaltigen Alken, worin jedes X unabhängig F oder Cl darstellt und zumindest ein X Cl ist;
einem durch die Formel (5) CH₂XCCl=CX₂ dargestellten chlorhaltigen Alken, worin jedes X unabhängig F oder Cl darstellt; und
einem durch die Formel (6) CHX₂CH=CX₂ dargestellten chlorhaltigen Alken, worin jedes X unabhängig F oder Cl darstellt,
wobei der Fluorierungskatalysator ein Katalysator ist, worin zumindest ein Metallelement M, ausgewählt aus der Gruppe, bestehend aus Indium, Gallium, Kobalt, Kupfer, Nickel, Zink und Aluminium, zusammen mit Chrom vorliegt, worin das molare Verhältnis von dem zumindest einem Metallelement M zu Chrom 96:4 bis 65:35 beträgt und worin das Metallelement M kristallines Nickelfluorid umfasst.

2. Verfahren gemäß Anspruch 1, worin der Fluorierungskatalysator ein Katalysator ist, worin Nickelfluorid zusammen mit Chromoxid vorliegt.

3. Verfahren gemäß irgendeinem der Ansprüche 1 bis 2, worin das molare Verhältnis von dem zumindest einem Metallelement M zu Chrom 90:10 bis 75:25 beträgt.

4. Verfahren gemäß irgendeinem der Ansprüche 1 bis 3, worin der Fluorierungskatalysator nicht auf einem Träger geträgert ist.

5. Verfahren gemäß irgendeinem der Ansprüche 1 bis 3, worin der Fluorierungskatalysator auf einem Träger geträgert ist, ausgewählt aus der Gruppe, bestehend aus Nickelfluorid, Chromfluorid, Aluminiumfluorid, fluoriertem Aluminiumoxid, fluorierter Aktivkohle und Graphit-Kohlenstoff.

6. Verfahren gemäß irgendeinem der Ansprüche 1 bis 5, worin der Fluorierungskatalysator eine spezifische Oberfläche von 30 m²/g oder größer aufweist.

7. Verfahren gemäß irgendeinem der Ansprüche 1 bis 6, worin die chlorhaltige Verbindung zumindest eine ist, ausgewählt aus der Gruppe, bestehend aus HCC-240db, HCO-1230xa, HCO-1230xf, HCFC-243db, HCFC-242dc, HCFO-1231xf, HCFO-1232xf und HCFO-1233xf, und worin das zu erhaltende fluorhaltige Olefin HCFO-1233xf oder HFO-1234yf ist.

8. Verfahren gemäß irgendeinem der Ansprüche 1 bis 7, worin die chlorhaltige Verbindung zumindest eine ist, ausgewählt aus der Gruppe, bestehend aus HCC-240fa, HCO-1230za, HCFC-243fa und HCFO-1233zd, und das zu erhaltende fluorhaltige Olefin HCFO-1233zd oder HFO-1234ze ist.

## Revendications

1. Procédé pour produire une oléfine contenant du fluor représentée par la formule (7) : CF₃CA=CHB dans laquelle l'un parmi A et B est F ou Cl et l'autre est H,
le procédé comprenant la réaction, en présence d'oxygène et d'un catalyseur de fluoration, de fluorure d'hydrogène anhydre avec au moins un composé contenant du chlore choisi dans le groupe constitué par
un alcane contenant du chlore représenté par la formule (1) CX₃CClYCH₂Z dans laquelle chaque X représente indépendamment F ou Cl, Y est H ou F, lorsque Y est H, Z est Cl ou F, et lorsque Y est F, Z est H ;
un alcane contenant du chlore représenté par la formule (2) CX₃CH₂CHX₂ dans laquelle chaque X représente indépendamment F ou Cl, et au moins un X est Cl ;
un alcène contenant du chlore représenté par la formule (3) CX₃CCl=CH₂ dans laquelle chaque X représente indépendamment F ou Cl ;
un alcène contenant du chlore représenté par la formule (4) CX₃CH=CHX dans laquelle chaque X représente indépendamment F ou Cl, et au moins un X est Cl ;
un alcène contenant du chlore représenté par la formule (5) CH₂XCCl=CX₂ dans laquelle chaque X représente indépendamment F ou Cl ; et
un alcène contenant du chlore représenté par la formule (6) CHX₂CH=CX₂ dans laquelle chaque X représente indépendamment F ou Cl,
le catalyseur de fluoration étant un catalyseur dans lequel au moins un élément métallique M choisi dans le groupe constitué par l'indium, le gallium, le cobalt, le cuivre, le nickel, le zinc et l'aluminium est présent conjointement avec le chrome, dans lequel le rapport molaire de l'au moins un élément métallique M avec le chrome est de 96/4 à 65/35, et dans lequel l'élément métallique M comprend du fluorure de nickel cristallin.

2. Procédé selon la revendication 1, dans lequel le catalyseur de fluoration est un catalyseur dans lequel du fluorure de nickel est présent conjointement avec de l'oxyde de chrome.

3. Procédé selon l'une quelconque des revendications 1 à 2, dans lequel le rapport molaire de l'au moins un élément métallique M avec le chrome est de 90/10 à 75/25.

4. Procédé selon l'une quelconque des revendications 1 à 3, dans lequel le catalyseur de fluoration n'est pas supporté sur un support.

5. Procédé selon l'une quelconque des revendications 1 à 3, dans lequel le catalyseur de fluoration est supporté sur un support choisi dans le groupe constitué par le fluorure de nickel, le fluorure de chrome, le fluorure d'aluminium, l'alumine fluorée, le carbone activé fluoré et le carbone graphitique.

6. Procédé selon l'une quelconque des revendications 1 à 5, dans lequel le catalyseur de fluoration a une surface spécifique de 30 m²/g ou plus.

7. Procédé selon l'une quelconque des revendications 1 à 6, dans lequel le composé contenant du chlore est au moins un élément choisi dans le groupe constitué par HCC-240db, HCO-1230xa, HCO-1230xf, HCFC-243db, HCFC-242dc, HCFO-1231xf, HCFO-1232xf et HCFO-1233xf, et l'oléfine contenant du fluor à obtenir est HCFO-1233xf ou HFO-1234yf.

8. Procédé selon l'une quelconque des revendications 1 à 7, dans lequel le composé contenant du chlore est au moins un élément choisi dans le groupe constitué par HCC-240fa, HCO-1230za, HCFC-243fa et HCFO-1233zd, et l'oléfine contenant du fluor à obtenir est HCFO-1233zd ou HFO-1234ze.
